# EUROPEAN PATENT SPECIFICATION

(11) **EP 3 386 486 B1**
(45) Date of publication and mention of the grant of the patent: **04.12.2019**
(21) Application number: 16809332.6
(22) Date of filing: 05.12.2016
(51) Int. Cl.: A61K 9/16

(54) **ENCAPSULATING AGENT WITH IMPROVED PROPERTIES ADAPTED FOR CELL ENCAPSULATION**
VERKAPSELUNGSMITTEL MIT VERBESSERTEN EIGENSCHAFTEN ZUR ZELLVERKAPSELUNG
AGENT D'ENCAPSULATION AYANT DES PROPRIÉTÉS AMÉLIORÉES ADAPTÉ POUR L'ENCAPSULATION DE CELLULES

(30) Priority: 08.12.2015 LU 92895
(43) Date of publication of application: 17.10.2018
(73) Proprietor: Luxembourg Institute of Science and Technology (LIST), 4362 Esch-sur-Alzette (LU)
(72) Inventor: SCHLEEH, Thomas, 54338 Schweich (DE)
(74) Representative: Lecomte & Partners
(86) International application number: PCT/EP2016/079707
(87) International publication number: WO 2017/097688

(56) References cited:
- PLUEMSAB ET AL: "Synthesis and inclusion property of @a-cyclodextrin-linked alginate", POLYMER, ELSEVIER SCIENCE PUBLISHERS B.V, GB, vol. 46, no. 23, 14 November 2005 (2005-11-14), pages 9778-9783, XP005115553, ISSN: 0032-3861, DOI: 10.1016/J.POLYMER.2005.08.005
- "Cyclodextrin-linked alginate beads as supporting materials for Sphingomonas cloacae, a nonylphenol degrading bacteria", BIORESOURCE TECHNOLOGY, vol. 98, 18 October 2006 (2006-10-18), pages 2076-2081, XP002760216,
- NAKAUMA MAKOTO ET AL: "Calcium binding and calcium-induced gelation of sodium alginate modified by low molecular-weight polyuronate", FOOD HYDROCOLLOIDS, vol. 55, 18 November 2015 (2015-11-18), pages 65-76, XP029367550, ISSN: 0268-005X, DOI: 10.1016/J.FOODHYD.2015.10.021

## Description

### Technical field

The invention is directed to the field of encapsulating agent adapted for encapsulating cell or cells.

### Background art

An interpenetrating polymer network is a specific three-dimensional polymer network which comprises two or more networks that are at least partially interlaced on a molecular scale but not covalently bonded to each other and cannot be separated unless chemicals bonds are broken.

Microcapsules mean particles each comprising a matrix material having embedded therein a plurality of solid or liquid microparticles or solutes molecules. Microcapsules usually have a mean diameter of about 5 mm or smaller, e.g. between 1 mm and 0.05 mm, such as between 0.6 and 0.1 mm. They can also have a diameter e.g. between 2 mm and 0.01 mm, such as between 1.5 mm and 0.2 mm. Microcapsule can permanently or temporarily entrap substances like e.g. drugs, pesticides, dyes... Microcapsules can be designed in accordance with the chemical structure of a three-dimensional polymer network, and more specifically, in accordance with the chemical structure of an interpenetrating polymer network.

A hydrogel is a gel in which the swelling agent is water and may form a microcapsule. The network component of a hydrogel is usually a polymer.

A micro-sphere is a kind of microcapsule with a spherical shape without any membrane or any distinct outer layer.

A micro-bead is a polyethylene micro-sphere that is widely used in cosmetics as exfoliating agents and personal care products such as toothpaste, as well as biomedical and health science research, microscopy techniques, fluid visualization, fluid flow analysis, and process troubleshooting.

Polyuronic acids and their sodium salts, in particular alginic acid and sodium alginate, are known to present biocompatibility properties, are easy to process and are cheap. Moreover, alginate is a FDA approved compound. These components could be therefore used to make the microcapsule, the micro-sphere and/or the micro-beads.

Prior art patent document published US 6,960,617 B2 discloses hydrogels having improved elasticity and improved mechanical strength properties. The authors describe a hydrogel formulation comprising a strengthening agent and a primary polymer, forming thereby interpenetrating networks (IPNs). The strengthening agent can be a polysaccharide such as one or more selected from alginate and derivatives thereof, cyclodextrin (CD), dextran and derivatives thereof, among many others such as chitins, chitosan, cellulose, gums, lignins, pectins, saponins, deoxyribonucleic acids, ribonucleic acid, polypeptide, protein....A pharmaceutical composition in solid dosage form which comprises a pharmacologically effective dose of a drug and a strengthened hydrogel or superporous hydrogel is disclosed. The pharmaceutical composition is typically in tablet, capsule or particulate form and can be administered to a patient orally, mucosally, transdermally or similar way.

Cyclodextrins (CDs) are interesting macrocycles presenting an outer surface which is hydrophilic and an inner surface which is hydrophobic and which is able to entrap hydrophobic moieties.

In the field of microcapsules, the result of non-covalent host-guest interaction between CDs and polymers are named polymer inclusion complexes (PICs).

Liu Y. *et al.* have used α-CD as "artificial chaperones" to facilitate the self-assembly of a double hydrophilic block copolymer, poly(ethylene oxide)-b-poly(4-vinylpyridine) (PEO-b-P4VP), in the synthesis of polymeric vesicles (Liu Y. et al., Polymer, 2009, 50, 855-859). At a pH of 4, the polymer was first complexed with α-CD and allowed to self-assemble into metastable micelles. The P4VP corona was then ionically cross-linked with poly(ethylene oxide)-b-poly(acrylic acid) (PEO-b-PAA). Through dialysis, the α-CD was removed from the system to produce vesicles, and, when no α-CD was used, the system formed cross-linked micelles.

Supramolecular structures consisting in an inclusion complex of linear poly(ethyleneimine) with α-CD and γ-CD have been reported (Choi H. S. et al., Macromol., 2004, 37, 6705-6710).

Double-axle intrusion (DI) system using γ-CD and linear diblock copolymers have been reported (Joung Y.-K. et al., Adv. Mater., 2007, 19 396-400). The rheological properties in response to pH changes of those particular systems can be modulated.

Pluemsab W. et al. have used the synthesis and inclusion property of α-cyclodextrin-linked alginate to form inclusion complex with p-nitrophenol as a guest compound.

A major drawback of the known system is in regard with the long-term stability of the microcapsules, micro-sphere, and/or micro-bead. Under the influence of the external conditions, such an alkaline media, those encapsulating agent collapse and disintegrate. Therefore, if cells are entrapped in such an agent, itself implanted in the human body, upon collapse of the entrapping agent, the cells are suddenly exposed to the external conditions and they died, without providing enough health benefit to the patient.

### Summary of invention

### Technical Problem

The invention has for technical problem to provide an interpenetrating polymer network adapted for cell encapsulation which overcomes the above mentioned drawbacks, in particular to provide such encapsulating agent which is stable in the long-term. It is indeed important for the cells present in the inner core of the microcapsule to survive and to work for long period of time in order to allow them to provide sufficient beneficial effects to the living body in which the microcapsules are located.

### Technical solution

The invention has for first object a three-dimensional polymer network according to claim 1 for encapsulating a pharmaceutical ingredient, said polymer network comprising (a) at least one first polymer; and (b) at least one cross-linking agent. Said three-dimensional polymer network is remarkable in that said at least one first polymer comprises a first polyuronate derivative, said first polyuronate derivative being modified with a hydrophobic moiety; and in that said at least one cross-linking agent is calcium chloride and/or a cyclodextrin derivative.

In a preferred embodiment, said three-dimensional polymer network further comprises a second polymer, said second polymer comprising preferentially a second polyuronate derivative, said second polyuronate derivative being preferentially unmodified.

In a preferred embodiment, said cyclodextrin derivative is selected from cyclodextrin, polymerized cyclodextrin, cyclodextrin modified with alginate with a degree of substitution equal to 1 or cyclodextrin modified with alginate with a degree of substitution inferior to 1.

In a preferred embodiment, said first polyuronate derivative has a degree of substitution equal to 1 or a degree of substitution inferior to 1.

In a preferred embodiment, said three-dimensional polymer network comprises a first polymer being a first polyuronate derivative modified with a hydrophobic moiety with a degree of substitution equal to 1, a second polymer being a second polyuronate derivative which is unmodified, and a cross-linking agent being calcium chloride and cyclodextrin, thereby forming an interpenetrating polymer network.

In a preferred embodiment, said three-dimensional polymer network comprises a first polymer being a first polyuronate derivative modified with a hydrophobic moiety with a degree of substitution equal to 1, a second polymer being a second polyuronate derivative which is unmodified, and a cross-linking agent being calcium chloride and polymerized cyclodextrin, thereby forming an interpenetrating polymer network.

In a preferred embodiment, said three-dimensional polymer network comprises a first polymer being a first polyuronate derivative modified with a hydrophobic moiety with a degree of substitution equal to 1, a second polymer being a second polyuronate derivative which is unmodified, and a cross-linking agent being calcium chloride and cyclodextrin modified with alginate with a degree of substitution equal to 1, thereby forming an interpenetrating polymer network.

In a preferred embodiment, said first polyuronate derivative and second polyuronate derivative are one of the derivative selected from mannuronate derivatives, guluronate derivatives, alginate derivatives, pectin derivatives, iduronate derivatives, galacturonate derivatives, lignin derivatives and/or any combination thereof.

In a preferred embodiment, said hydrophobic moiety modifying said first polyuronate derivative is selected from an alkyl moiety, a phenyl alkyl moiety, a fluoroalkane and/or any other hydrophobic derivatives.

In a preferred embodiment, said hydrophobic moiety modifying said first polyuronate derivative is covalently bounded to said first polyuronate derivative by an amide moiety, an ester moiety, a thioester moiety, a phosphonate moiety, an ether moiety, a thioether moiety, an imine moiety, or any other chemical group.

In a preferred embodiment, said three-dimensional polymer network encapsulates at least one pharmaceutical ingredient, said pharmaceutical ingredient being preferentially cells, more preferentially Langerhans islets.

The invention has for second object an encapsulating agent adapted for encapsulation of pharmaceutical ingredient. Said encapsulating agent is remarkable in that it comprises a three-dimensional polymer network according to the first object of the invention.

In a preferred embodiment, said encapsulating agent is under the form of a microcapsule, a micro-sphere or a micro-bead.

The invention has for third object an encapsulating agent for use as a medicament, preferentially for use in the treatment of cancer, diabetes and/or Parkinson disease, remarkable in that said encapsulating agent is in accordance with the preferred embodiment of the second object of the invention.

The invention has for fourth object a process for making a three-dimensional polymer network, said process comprising the steps of (a) preparing an aqueous solution of at least one first polymer; (b) preparing an aqueous solution of at least one cross-linking agent; (c) mixing together under stirring the aqueous solutions so obtained in steps (a) and (b); and (d) after at least 30 minutes, filtering out the three-dimensional polymer network. Said process is remarkable in that said three-dimensional polymer network is in accordance with the three-dimensional polymer network of the first object of the invention.

### Advantages of the invention

The invention is particularly interesting in that the encapsulating agents demonstrate an improved long-term stability and adjustable properties. The encapsulating agent of the present invention has a mesh size of the interpenetrating polymer network which is flexible because the hydrophobic groups present on the water-soluble polyelectrolyte are only entrapped or caged into the cyclodextrin inner cavity through supramolecular interaction. As this is not a covalent bonding between these two entities, the interpenetrating polymer network composing the encapsulating agent is flexible. Such kind of polymer network is therefore easily expandable and shrinkable.

### Brief description of the drawings

Figure 1: Scheme of three-dimensional polymer network in accordance with the present invention.
Figure 2: Scheme of the crosslinking of two hydrophobic groups provided by a cyclodextrin derivative.
Figure 3: Scheme of the crosslinking of one hydrophobic group provided by a polymeric cyclodextrin derivative.
Figure 4: Generic chemical structure of the compounds used in the present invention.

### Description of an embodiment

In order to provide an encapsulating agent able to encapsulate a cell, it is interesting to achieve such encapsulating agent which is not only very stable but also very flexible, *i.e.* which can easily swell and shrink. These properties will confer protection to the encapsulated cell.

The three-dimensional polymer network according to the present invention, or the specific interpenetrating polymer network, comprises a first polymer network formed by an ionic cross-linked hydrogel formed by a polyuronate derivative. A polyuronate derivative is a water-soluble polyelectrolyte with a polysaccharide structure, and can be chosen among mannuronate derivatives, guluronate derivatives, alginate derivatives, pectin derivatives, iduronate derivatives, galacturonate derivatives, lignin derivatives and/or any combinations thereof.

Preferentially, alginate derivatives are used.

Said polyuronate derivative, in particular said alginate derivative, is chemically modified with at least one hydrophobic moiety, which may be an alkyl moiety, a phenyl alkyl moiety, a fluoroalkane and/or any other hydrophobic derivatives.

Said hydrophobic moiety is covalently bounded to the polyuronate derivatives by an amide moiety. Other chemical functionalities can also be employed, such as an ester moiety, a thioester moiety, a phosphonate moiety, an ether moiety, a thioether moiety, an imine moiety, or any other.

Said polyuronate derivatives can also be chemically functionalized with 6-monodeoxy-6-monoamino-β-cyclodextrin hydrochloride.

The polyuronate derivatives are cross-linked with each other through a multivalent ion, preferentially Ca⁺⁺. This is achieved by adding calcium chloride during the process of making such an interpenetrating polymer network.

The three-dimensional polymer network according to the present invention, or the specific interpenetrating polymer network, comprises a second polymer network formed by cyclodextrin moieties or by derivatives thereof such as polymerized cyclodextrins. Such polymerized cyclodextrin is formed through chemical connection of cyclodextrin moieties with epichlorhydrin.

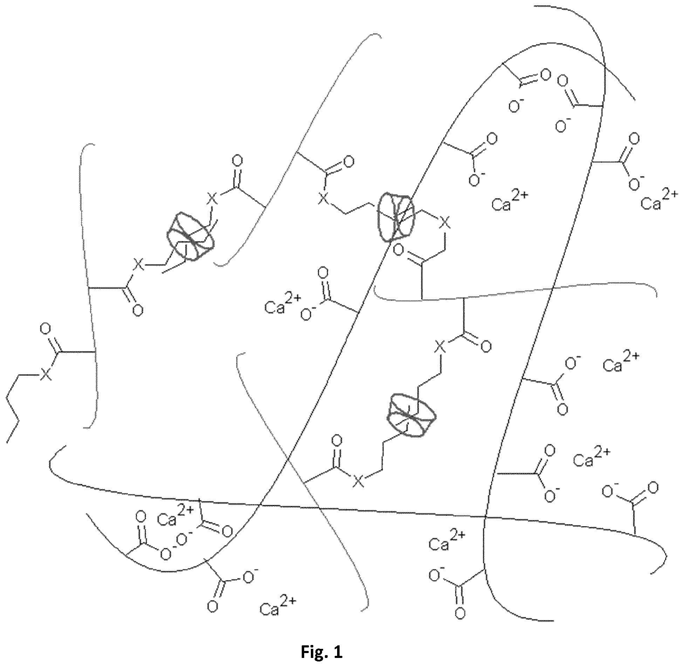

Figure 1 schematically represents a three-dimensional polymer network in accordance with the present invention.

The cyclodextrins are a class of compound which presents an outer hydrophilic surface and an inner hydrophobic cavity. Cyclodextrins are distinguished according to the number of saccharide rings which correspond to a specific cavity diameter. For instance, the cavity diameter of α-cydodextrin is 4.7Å-5.3Å, the cavity diameter of β-cydodextrin is 6.0Å-6.5Å, and the cavity diameter of γ-cyclodextrin is 7.5Å-8.3Å. This hollow hydrophobic cavity can interact with hydrophobic compounds through weak interaction, *i.e.,* Van der Waals interactions.

The cyclodextrin derivatives can thus host the hydrophobic moiety which is present on the polyuronate derivatives.

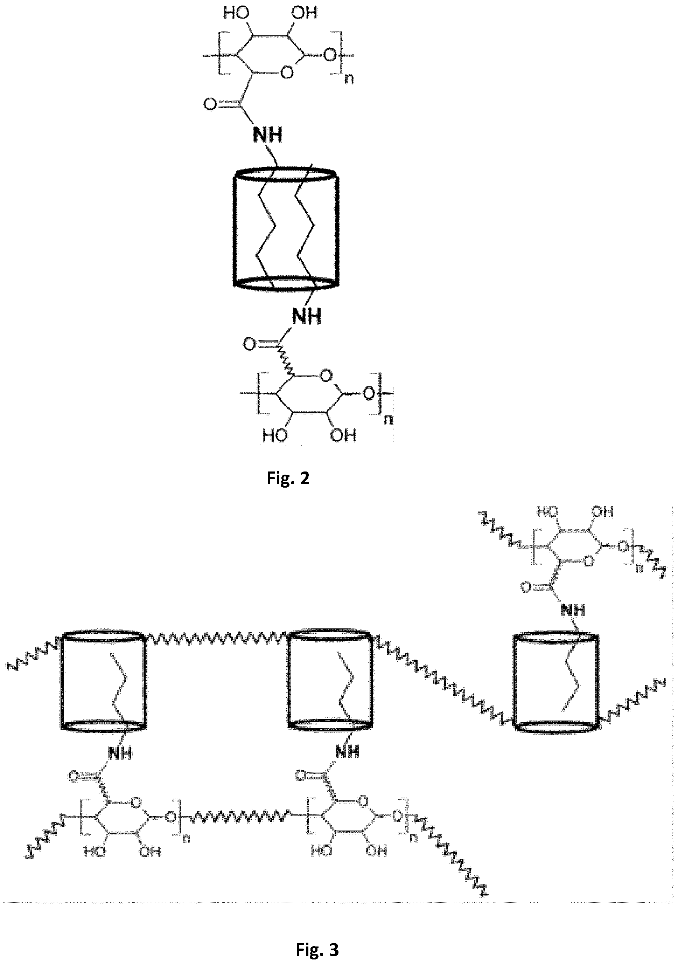

These supramolecular interactions, in particular these Van der Waals interactions, which are showed either on figure 2 or on figure 3, allow a very high flexibility to the interpenetrating polymer network.

In figure 2, the cyclodextrin derivative cross-links two hydrophobic groups.

In figure 3, the cyclodextrin derivative (*i.e.* a polymeric cyclodextrin) cross-links only one hydrophobic group.

This allows to the whole structure to be kept together, and therefore, to confer protection to any objects, like cell or cells, which are entrapped or encapsulated inside the three-dimensional polymer network schematically depicted in figure 1 (these objects are not shown).

Table 1 indicates an overview of the polymers and crosslinking agents which can be combined to furnish the three-dimensional polymer network:

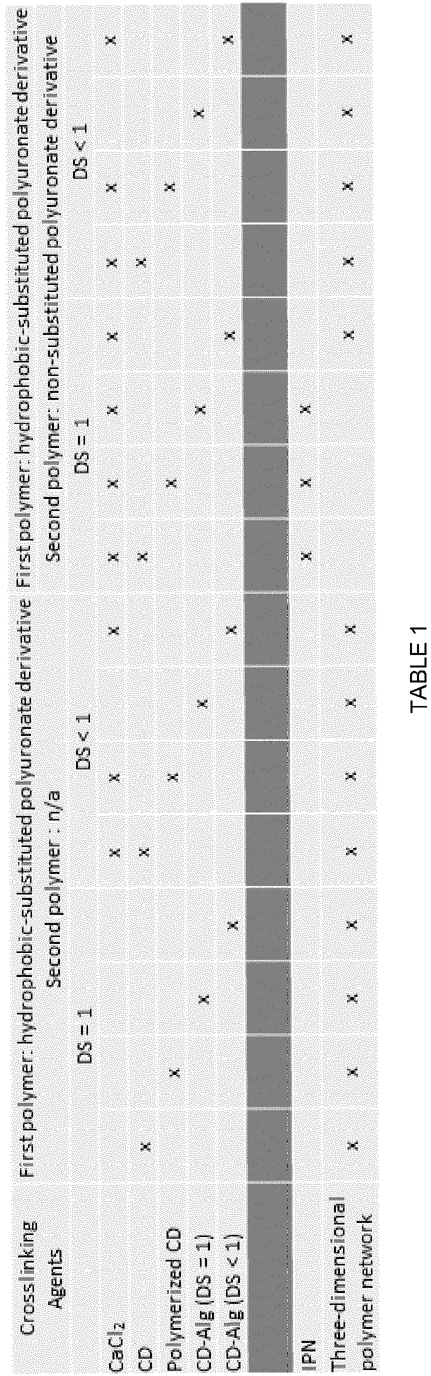

The crosslinking agents can thus be chosen among calcium chloride (CaCl₂), cyclodextrin derivatives (CD), polymerized cyclodextrin (p-CD) and/or any combination thereof. Among the cyclodextrin derivatives, a preferred derivative is a cyclodextrin modified with alginate. The degree of substitution, which corresponds to the (average) number of substituent groups attached per base unit or per monomeric unit can be equal to 1 or be inferior to 1.

The three-dimensional polymer network according to the present invention, or the specific interpenetrating polymer network, may comprise a first polymer and a second polymer, or only one polymer.

Preferentially, a hydrophobic substituted polyuronic acid salt (a polyuronate derivative modified with a hydrophobic moiety) is used as first polymer, or as said one polymer in case where only one polymer is used.

In case where two polymers are used, a hydrophobic substituted polyuronic acid salt (a polyuronate derivative modified with a hydrophobic moiety) is used as first polymer and a polyuronic acid salt which is unsubstituted (a polyuronate derivative which is unmodified) is used as second polymer.

The three-dimensional polymer network containing hydrophobic substituted polyuronic salt (DS=1) and cyclodextrin has been designed.

The three-dimensional polymer network containing hydrophobic substituted polyuronic salt (DS=1) and polymerized cyclodextrin has been designed.

The three-dimensional polymer network containing hydrophobic substituted polyuronic salt (DS=1) and a cyclodextrin modified with alginate (DS=1) has been designed.

The three-dimensional polymer network containing hydrophobic substituted polyuronic salt (DS=1) and a cyclodextrin modified with alginate (DS<1) has been designed.

The three-dimensional polymer network containing hydrophobic substituted polyuronic salt (DS<1), calcium chloride and cyclodextrin has been designed.

The three-dimensional polymer network containing hydrophobic substituted polyuronic salt (DS<1), calcium chloride and polymerized cyclodextrin has been designed.

The three-dimensional polymer network containing hydrophobic substituted polyuronic salt (DS<1) and cyclodextrin modified with alginate (DS=1) has been designed.

The three-dimensional polymer network containing hydrophobic substituted polyuronic salt (DS<1), calcium chloride and a cyclodextrin modified with alginate (DS<1) has been designed.

The three-dimensional polymer network containing hydrophobic substituted polyuronic salt as first polymer (DS=1), an unsubstituted polyuronic salt as second polymer, calcium chloride and cyclodextrin has been designed. This three-dimensional polymer network has been characterized as forming an interpenetrating polymer network.

The three-dimensional polymer network containing hydrophobic substituted polyuronic salt as first polymer (DS=1), an unsubstituted polyuronic salt as second polymer, calcium chloride and polymerized cyclodextrin has been designed. This three-dimensional polymer network has been characterized as forming an interpenetrating polymer network.

The three-dimensional polymer network containing hydrophobic substituted polyuronic salt as first polymer (DS=1), an unsubstituted polyuronic salt as second polymer, calcium chloride and a cyclodextrin modified with alginate (DS=1) has been designed. This three-dimensional polymer network has been characterized as forming an interpenetrating polymer network.

The three-dimensional polymer network containing hydrophobic substituted polyuronic salt as first polymer (DS=1), an unsubstituted polyuronic salt as second polymer, calcium chloride and cyclodextrin modified with alginate (DS<1) has been designed.

The three-dimensional polymer network containing hydrophobic substituted polyuronic salt as first polymer (DS<1), an unsubstituted polyuronic salt as second polymer, calcium chloride, and cyclodextrin has been designed.

The three-dimensional polymer network containing hydrophobic substituted polyuronic salt as first polymer (DS<1), an unsubstituted polyuronic salt as second polymer, calcium chloride and polymerized cyclodextrin has been designed.

The three-dimensional polymer network containing hydrophobic substituted polyuronic salt as first polymer (DS<1), an unsubstituted polyuronic salt as second polymer and cyclodextrin modified with alginate (DS=1) has been designed.

The three-dimensional polymer network containing hydrophobic substituted polyuronic salt as first polymer (DS<1), an unsubstituted polyuronic salt as second polymer, calcium chloride and cyclodextrin modified with alginate (DS<1) has been designed.

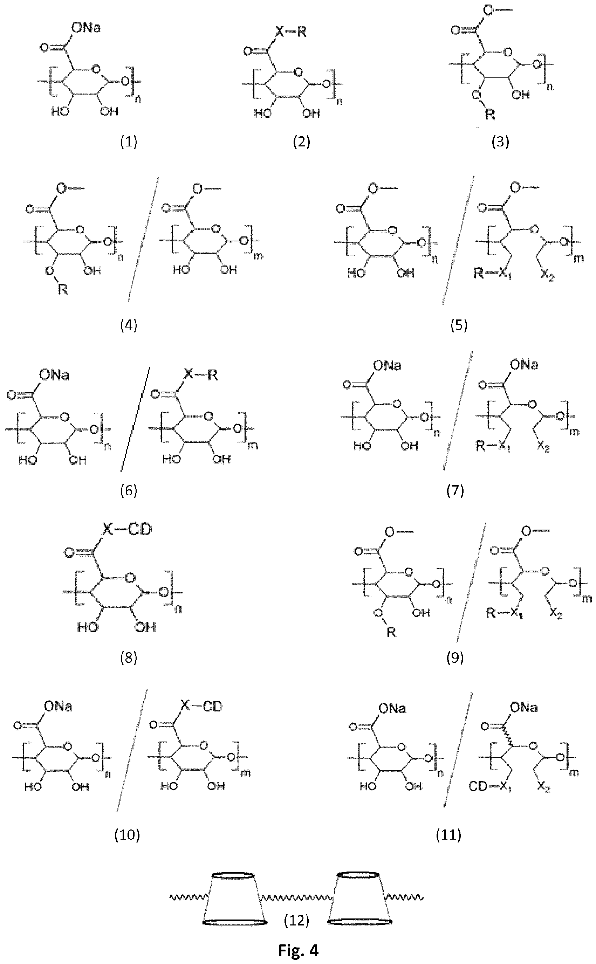

Figure 4 indicates the generic structure of the compounds which have been used in the course of the present invention.

The compound of formula 1 is the sodium salt of the polyuronic acids. Alternatively, the potassium salt (not shown) can be used.

The compounds of formulas 2, 3, 4 and 5 are the hydrophobic modified polyuronic acids lacking of carboxylic acid moieties.

The compounds of formulas 6 and 7 are the hydrophobic modified sodium salt of the polyuronic acids.

The compounds of formulas 8, 9, 10 and 11 are the polyuronic acids modified with the cyclodextrin (CD).

Finally, the compound 12 schematically represents a polymerized cyclodextrin.

In order to design the three-dimensional polymer network according to the present invention, or the specific interpenetrating polymer network, the following procedure has been applied.

The preparation of an aqueous solution of the first polymer is performed.

If a second polymer has to be incorporated to the polymer network, the preparation of an aqueous solution of the second polymer is performed.

A cyclodextrin derivative must be added to the aqueous solution of the polymer.

Both said aqueous solution are then mixed upon each other and added to a hardening aqueous solution of calcium chloride. After gentle stirring, the microcapsule becomes harder and can be filtered out from the solution.

The three-dimensional polymer network according to the present invention, or the specific interpenetrating polymer network, is particular in that its building blocks, *i.e.* the cross-linking moieties, confer an improved protection to any active ingredients which are entrapped or encapsulated in the inner core of the network.

Indeed, once the three-dimensional polymer network or the interpenetrating polymer network starts, for any reason(s), to disintegrate, the cyclodextrin moieties start to cross-link with other hydrophobic moieties present in their close surroundings. Therefore, the whole system can be maintained as such, and the entrapped or encapsulated objects, e.g. cells, can stay protected from the external environment.

It has been in particular shown that in such a system the cells are still alive at a level of pH up to 9.5, in presence of a high concentration of sodium ion.

Examples of cells which can be encapsulated are the Langerhans islets which comprise hormone-producing cells. The size of such islets is comprised between 200 µm and 500 µm.

The three-dimensional polymer network or the interpenetrating polymer network as described above is the main component of an encapsulating agent adapted for cell encapsulation or adapted for cells encapsulation. Said encapsulating agent is either a microcapsule or a micro-sphere or a micro-bead.

The entrapped cells in the three-dimensional polymer network or in the interpenetrating polymer network (and subsequently in the encapsulating agent) is/are cell(s) able to be fed and able to secrete the proteins and/or the compounds used to treat disease, in particular burdensome diseases, such as cancer, diabetes, Parkinson's disease and/or any other.

These encapsulating agents can therefore act as a medicament and can further be implanted within the body of a living being, preferentially a human being. They can be grafted or simply inserted under the skin. They can also be swallowed by a subject.

Example of design of three-dimensional polymer network.

A microcapsule has been designed comprising alginic acid butyl amide sodium salt as first polymer (DS_{amide} = 0.41), alginic acid sodium salt (compound of formula 1 on figure 2) as second polymer, calcium chloride and β-cyclodextrin as cross-liking agents.

50 mL of a solution comprising 1.66 % (w/v) aqueous alginic acid solution containing 0.82 % (w/v) β-cyclodextrin are prepared (solution 1).

50 mL of a solution comprising 2 % (w/v) aqueous alginic acid sodium salt solution are prepared (solution 2).

Both solutions 1 and 2 are mixed together, thereby forming a polymer aqueous solution.

Then the desired active ingredient is added in a predetermined amount, *e.g.* 1000 - 5000 cells/mL. Polysorbate as surfactant can be added in order to improve the homogeneity of the dissolved compounds.

1 L of a 0.1 M aqueous CaCl₂ solution is prepared as a hardening bath. Then, the hardening bath is poured into a crystallizer dish (*e.g*. 2 - 3 cm filling height) and is stirred gently with a magnetic bar. The polymer solution is then dropped into the hardening bath with an encapsulator device or a microsyringe and the hardening of the microcapsules lasts at least 30 minutes under gentle stirring.

The microcapsules are then filtered out from the solution.

## Claims

1. Three-dimensional polymer network for encapsulating a pharmaceutical ingredient, said polymer network comprising
a) at least one first polymer; and
b) at least one cross-linking agent;
**characterized in that**
said at least one first polymer comprises a first polyuronate derivative, said first polyuronate derivative being modified with a hydrophobic moiety; and **in that** said at least one cross-linking agent is calcium chloride and a cyclodextrin derivative.

2. Three-dimensional polymer network according to claim 1, **characterized in that** said three-dimensional polymer network further comprises a second polymer, said second polymer comprising preferentially a second polyuronate derivative, said second polyuronate derivative being preferentially unmodified.

3. Three-dimensional polymer network according to any one of claims 1-2, **characterized in that** said cyclodextrin derivative is selected from cyclodextrin, polymerized cyclodextrin, cyclodextrin modified with alginate with a degree of substitution equal to 1 or cyclodextrin modified with alginate with a degree of substitution inferior to 1.

4. Three-dimensional polymer network according to any one of claims 1-3, **characterized in that** said first polyuronate derivative has a degree of substitution equal to 1 or a degree of substitution inferior to 1.

5. Three-dimensional polymer network according to claim 4, **characterized in that** said three-dimensional polymer network comprises
a) a first polymer being a first polyuronate derivative modified with a hydrophobic moiety with a degree of substitution equal to 1,
b) a second polymer being a second polyuronate derivative which is unmodified, and
c) a cross-linking agent being calcium chloride and cyclodextrin,
thereby forming an interpenetrating polymer network.

6. Three-dimensional polymer network according to claim 4, **characterized in that** said three-dimensional polymer network comprises
a) a first polymer being a first polyuronate derivative modified with a hydrophobic moiety with a degree of substitution equal to 1,
b) a second polymer being a second polyuronate derivative which is unmodified, and
c) a cross-linking agent being calcium chloride and polymerized cyclodextrin,
thereby forming an interpenetrating polymer network.

7. Three-dimensional polymer network according to claim 4, **characterized in that** said three-dimensional polymer network comprises
a) a first polymer being a first polyuronate derivative modified with a hydrophobic moiety with a degree of substitution equal to 1,
b) a second polymer being a second polyuronate derivative which is unmodified, and
c) a cross-linking agent being calcium chloride and cyclodextrin modified with alginate with a degree of substitution equal to 1,
thereby forming an interpenetrating polymer network.

8. Three-dimensional polymer network according to any one of claims 1-7, **characterized in that** said first polyuronate derivative and second polyuronate derivative are one of the derivative selected from mannuronate derivatives, guluronate derivatives, alginate derivatives, pectin derivatives, iduronate derivatives, galacturonate derivatives, lignin derivatives and/or any combination thereof.

9. Three-dimensional polymer network according to any one of claims 1-8, **characterized in that** said hydrophobic moiety modifying said first polyuronate derivative is selected from an alkyl moiety, a phenyl alkyl moiety, a fluoroalkane and/or any other hydrophobic derivatives.

10. Three-dimensional polymer network according to any one of claims 1-9, **characterized in that** said hydrophobic moiety modifying said first polyuronate derivative is covalently bounded to said first polyuronate derivative by an amide moiety, an ester moiety, a thioester moiety, a phosphonate moiety, an ether moiety, a thioether moiety, an imine moiety, or any other chemical group.

11. Three-dimensional polymer network according to any one of claims 1-10, **characterized in that** said three-dimensional polymer network encapsulates at least one pharmaceutical ingredient, said pharmaceutical ingredient being preferentially cells, more preferentially Langerhans islets.

12. Encapsulating agent adapted for encapsulation of pharmaceutical ingredient, **characterized in that** said encapsulating agent comprises a three-dimensional polymer network according to any one of claims 1-11.

13. Encapsulating agent according to claim 12, **characterized in that** said encapsulating agent is under the form of a microcapsule, a micro-sphere or a micro-bead.

14. Encapsulating agent for use as a medicament, preferentially for use in the treatment of cancer, diabetes and/or Parkinson disease, **characterized in that** said encapsulating agent is in accordance with claim 13.

15. Process for making a three-dimensional polymer network, said process comprising the following steps:
a) preparing an aqueous solution of at least one first polymer;
b) preparing an aqueous solution of at least one cross-linking agent;
c) mixing together under stirring the aqueous solutions so obtained in steps (a) and (b); and
d) after at least 30 minutes, filtering out the three-dimensional polymer network;
**characterized in that** said three-dimensional polymer network is in accordance with the three-dimensional polymer network of any one of claims 1-11.

## Patentansprüche

1. Dreidimensionales Polymernetzwerk zum Einkapseln eines pharmazeutischen Bestandteils, wobei das besagte Polymernetzwerk umfasst
a) zumindest ein erstes Polymer; und
b) zumindest ein Vernetzungsmittel; **gekennzeichnet dadurch, dass** das besagte zumindest eine erste Polymer ein erstes Polyuronatderivat umfasst, wobei das besagte erste Polyuronatderivat mit einer hydrophoben Gruppe modifiziert ist; und dass das besagte zumindest eine Vernetzungsmittel Calciumchlorid und ein Cyclodextrinderivat ist.

2. Dreidimensionales Polymernetzwerk gemäß Anspruch 1, **dadurch gekennzeichnet, dass** das besagte dreidimensionale Polymernetzwerk weiter ein zweites Polymer umfasst, wobei das besagte zweite Polymer vorzugsweise ein zweites Polyuronatderivat umfasst, wobei das besagte zweite Polyuronatderivat vorzugsweise unmodifiziert ist.

3. Dreidimensionales Polymernetzwerk gemäß einem der Ansprüche 1-2, **dadurch gekennzeichnet, dass** das besagte Cyclodextrinderivat ausgewählt ist aus Cyclodextrin, polymerisiertem Cyclodextrin, mit Alginat modifiziertem Cyclodextrin mit einem Substitutionsgrad gleich 1 oder mit Alginat modifiziertem Cyclodextrin mit einem Substitutionsgrad unter 1.

4. Dreidimensionales Polymernetzwerk gemäß einem der Ansprüche 1-3, **dadurch gekennzeichnet, dass** das besagte erste Polyuronatderivat einen Substitutionsgrad gleich 1 oder einen Substitutionsgrad unter 1 hat.

5. Dreidimensionales Polymernetzwerk gemäß Anspruch 4, **dadurch gekennzeichnet, dass** das besagte dreidimensionale Polymernetzwerk umfasst
a) ein erstes Polymer, das ein erstes Polyuronatderivat modifiziert mit einer hydrophoben Gruppe mit einem Substitutionsgrad gleich 1 ist,
b) ein zweites Polymer, das ein zweites Polyuronatderivat ist, das unmodifiziert ist, und
c) ein Vernetzungsmittel, das Calciumchlorid und Cyclodextrin ist,
und dadurch ein interpenetrierendes Polymernetzwerk bildet.

6. Dreidimensionales Polymernetzwerk gemäß Anspruch 4, **dadurch gekennzeichnet, dass** das besagte dreidimensionale Polymernetzwerk umfasst
a) ein erstes Polymer, das ein erstes Polyuronatderivat modifiziert mit einer hydrophoben Gruppe mit einem Substitutionsgrad gleich 1 ist,
b) ein zweites Polymer, das ein zweites Polyuronatderivat ist, das unmodifiziert ist, und
c) ein Vernetzungsmittel, das Calciumchlorid und polymerisiertes Cyclodextrin ist, und dadurch ein interpenetrierendes Polymernetzwerk bildet.

7. Dreidimensionales Polymernetzwerk gemäß Anspruch 4, **dadurch gekennzeichnet, dass** das besagte dreidimensionale Polymernetzwerk umfasst
a) ein erstes Polymer, das ein erstes Polyuronatderivat modifiziert mit einer hydrophoben Gruppe mit einem Substitutionsgrad gleich 1 ist,
b) ein zweites Polymer, das ein zweites Polyuronatderivat ist, das unmodifiziert ist, und
c) ein Vernetzungsmittel, das Calciumchlorid und mit Alginat modifiziertes Cyclodextrin mit einem Substitutionsgrad gleich 1 ist,
und dadurch ein interpenetrierendes Polymernetzwerk bildet.

8. Dreidimensionales Polymernetzwerk gemäß einem der Ansprüche 1-7, **dadurch gekennzeichnet, dass** das besagte erste Polyuronatderivat und zweite Polyuronatderivat ein Derivat sind ausgewählt aus Mannuronatderivaten, Guluronatderivaten, Alginatderivaten, Pektinderivaten, Iduronatderivaten, Galacturonatderivaten, Ligninderivaten und/oder jeder Kombination davon.

9. Dreidimensionales Polymernetzwerk gemäß einem der Ansprüche 1-8, **dadurch gekennzeichnet, dass** die besagte hydrophobe Gruppe, die das besagte erste Polyuronatderivat modifiziert, ausgewählt ist aus einer Alkylgruppe, einer Phenylalkylgruppe, einem Fluoralkan und/oder anderen hydrophoben Derivaten.

10. Dreidimensionales Polymernetzwerk gemäß einem der Ansprüche 1-9, **dadurch gekennzeichnet, dass** die besagte hydrophobe Gruppe, die das besagte erste Polyuronatderivat modifiziert, kovalent gebunden ist an das besagte erste Polyuronatderivat durch eine Amidgruppe, eine Estergruppe, eine Thioestergruppe, eine Phosphonatgruppe, eine Ethergruppe, eine Thioethergruppe, eine Imingruppe oder irgendeine andere chemische Gruppe.

11. Dreidimensionales Polymernetzwerk gemäß einem der Ansprüche 1-10, **dadurch gekennzeichnet, dass** das besagte dreidimensionale Polymernetzwerk zumindest einen pharmazeutischen Bestandteil einkapselt, wobei der besagte pharmazeutische Bestandteil vorzugsweise Zellen, noch bevorzugter Langerhans-Inseln sind.

12. Verkapselungsmittel, angepasst für die Verkapselung eines pharmazeutischen Bestandteils, **dadurch gekennzeichnet, dass** das besagte Verkapselungsmittel ein dreidimensionales Polymernetzwerk gemäß einem der Ansprüche 1 bis 11 umfasst.

13. Verkapselungsmittel gemäß Anspruch 12, **gekennzeichnet dadurch, dass** das besagte Verkapselungsmittel in der Form einer Mikrokapsel, einer Mikrosphäre oder einer Mikroperle ist.

14. Verkapselungsmittel zur Verwendung als Medikament, vorzugsweise zur Verwendung in der Behandlung von Krebs, Diabetis und/oder Parkinson, **dadurch gekennzeichnet, dass** das besagte Verkapselungsmittel gemäß Anspruch 13 ist.

15. Verfahren zum Herstellen eines dreidimensionalen Polymernetzwerks, wobei das besagte Verfahren die folgenden Schritte umfasst:
a) Vorbereiten einer wässrigen Lösung von zumindest einem ersten Polymer;
b) Vorbereiten einer wässrigen Lösung von zumindest einem Vernetzungsmittel;
c) Mischen der wässrigen Lösungen zusammen unter Rühren, die so in den Schritten (a) und (b) erhalten wurden; und
d) nach mindestens 30 Minuten, Herausfiltern des dreidimensionalen Polymernetzwerks;
**dadurch gekennzeichnet, dass** das besagte dreidimensionale Polymernetzwerk in Übereinstimmung ist mit dem dreidimensionalen Polymernetzwerk von einem der Ansprüche 1-11.

## Revendications

1. Réseau polymère en trois dimensions pour encapsuler un ingrédient pharmaceutique, ledit réseau polymère comprenant :
a) au moins un premier polymère ; et
b) au moins un agent de réticulation ;
**caractérisé en ce que**
ledit au moins un premier polymère comprend un premier dérivé de polyuronate, ledit premier dérivé de polyuronate étant modifié avec une fraction hydrophobe ; et **en ce que** ledit au moins un agent de réticulation représente du chlorure de calcium et un dérivé de cyclodextrine.

2. Réseau polymère en trois dimensions selon la revendication 1, **caractérisé en ce que** ledit réseau polymère en trois dimensions comprend en outre un second polymère, ledit second polymère comprenant de préférence un second dérivé de polyuronate, ledit second dérivé de polyuronate étant de préférence non modifié.

3. Réseau polymère en trois dimensions selon l'une quelconque des revendications 1 à 2, **caractérisé en ce que** ledit dérivé de cyclodextrine est choisi parmi la cyclodextrine, de la cyclodextrine polymérisée, de la cyclodextrine modifiée avec un alginate avec un degré de substitution égal à 1 ou de la cyclodextrine modifiée avec un alginate avec un degré de substitution inférieur à 1.

4. Réseau polymère en trois dimensions selon l'une quelconque des revendications 1 à 3, **caractérisé en ce que** ledit dérivé de polyuronate possède un degré de substitution égal à 1 ou un degré de substitution inférieur à 1.

5. Réseau polymère en trois dimensions selon la revendication 4, **caractérisé en ce que** ledit réseau polymère en trois dimensions comprend :
a) un premier polymère, à savoir un premier dérivé de polyuronate modifié avec une fraction hydrophobe avec un degré de substitution égal à 1 ;
b) un second polymère, à savoir un second dérivé de polyuronate qui est non modifié ; et
c) un agent de réticulation à savoir du chlorure de calcium et de la cyclodextrine ;
pour ainsi obtenir un réseau polymère interpénétrant.

6. Réseau polymère en trois dimensions selon la revendication 4, **caractérisé en ce que** ledit réseau polymère en trois dimensions comprend :
a) un premier polymère, à savoir un premier dérivé de polyuronate modifié avec une fraction hydrophobe avec un degré de substitution égal à 1 ;
b) un second polymère, à savoir un second dérivé de polyuronate qui est non modifié ; et
c) un agent de réticulation à savoir du chlorure de calcium et de la cyclodextrine polymérisée ;
pour ainsi obtenir un réseau polymère interpénétrant.

7. Réseau polymère en trois dimensions selon la revendication 4, **caractérisé en ce que** ledit réseau polymère en trois dimensions comprend :
a) un premier polymère, à savoir un premier dérivé de polyuronate modifié avec une fraction hydrophobe avec un degré de substitution égal à 1 ;
b) un second polymère, à savoir un second dérivé de polyuronate qui est non modifié ; et
c) un agent de réticulation à savoir du chlorure de calcium et de la cyclodextrine modifiée avec un alginate avec un degré de substitution égal à 1 ;
pour ainsi obtenir un réseau polymère interpénétrant.

8. Réseau polymère en trois dimensions selon l'une quelconque des revendications 1 à 7, **caractérisé en ce que** ledit premier dérivé de polyuronate et le second dérivé de polyuronate représentent un dérivé choisi parmi des dérivés de mannuronate, des dérivés de guluronate, des dérivés d'alginate, des dérivés de pectine, des dérivés d'iduronate, des dérivés de galacturonate, des dérivés de lignine et/ou l'une quelconque de leurs combinaisons.

9. Réseau polymère en trois dimensions selon l'une quelconque des revendications 1 à 8, **caractérisé en ce que** ladite fraction hydrophobe qui modifie ledit premier dérivé de polyuronate est choisie parmi une fraction alkyle, une fraction phénylalkyle, un fluoroalcane et/ou l'un quelconque de leurs dérivés hydrophobes.

10. Réseau polymère en trois dimensions selon l'une quelconque des revendications 1 à 9, **caractérisé en ce que** ladite fraction hydrophobe qui modifie ledit premier dérivé de polyuronate est lié de manière covalente audit premier dérivée de polyuronate via une fraction amide, une fraction ester, une fraction thioester, une fraction phosphate, une fraction éther, une fraction thioéther, une fraction imine ou n'importe quel autre groupe chimique.

11. Réseau polymère en trois dimensions selon l'une quelconque des revendications 1 à 10, **caractérisé en ce que** ledit réseau polymère en trois dimensions encapsule au moins un ingrédient pharmaceutique, ledit ingrédient pharmaceutique représentant de préférence des cellules, de manière plus référentielle, des îlots de Langerhans.

12. Agent d'encapsulation conçu pour l'encapsulation d'un ingrédient pharmaceutique, **caractérisé en ce que** ledit agent d'encapsulation comprend un réseau polymère en trois dimensions selon l'une quelconque des revendications 1 à 11.

13. Agent d'encapsulation selon la revendication 12, **caractérisé en ce que** ledit agent d'encapsulation se présente sous la forme d'une microcapsule, d'une microsphère ou d'une microbille.

14. Agent d'encapsulation pour son utilisation comme médicament, de préférence pour son utilisation dans le traitement du cancer, du diabète et/ou de la maladie de Parkinson, **caractérisé en ce que** ledit agent d'encapsulation est conforme à la revendication 13.

15. Procédé pour la préparation d'un réseau polymère en trois dimensions, ledit procédé comprenant les étapes suivantes consistant à :
a) préparer une solution aqueuse d'au moins un premier polymère ;
b) préparer une solution aqueuse d'au moins un agent de réticulation ;
c) mélanger l'une à l'autre tout en agitant les solutions aqueuses ainsi obtenues aux étapes (a) et (b) ; et
d) après au moins 30 minutes, retirer le réseau polymère en trois dimensions par filtration ;
**caractérisé en ce que** ledit réseau polymère en trois dimensions est conforme au réseau polymère en trois dimensions selon l'une quelconque des revendications 1 à 11.
